(19) Europäisches Patentamt / European Patent Office / Office européen des brevets

(11) **EP 1 513 499 B1**

(12) **EUROPEAN PATENT SPECIFICATION**

(45) Date of publication and mention
of the grant of the patent:
**18.01.2012 Bulletin 2012/03**

(51) Int Cl.:
*A61K 9/10* (2006.01) *A61K 31/192* (2006.01)

(21) Application number: **03737116.8**

(86) International application number:
**PCT/US2003/018922**

(22) Date of filing: **17.06.2003**

(87) International publication number:
**WO 2003/105804 (24.12.2003 Gazette 2003/52)**

(54) **IBUPROFEN SUSPENSION**

IBUPROFEN-SUSPENSION

SUSPENSION D'IBUPROFENE

(84) Designated Contracting States:
**AT BE BG CH CY CZ DE DK EE ES FI FR GB GR HU IE IT LI LU MC NL PT RO SE SI SK TR**

(30) Priority: **17.06.2002 US 388734 P**

(43) Date of publication of application:
**16.03.2005 Bulletin 2005/11**

(73) Proprietor: **TARO PHARMACEUTICALS U.S.A., INC.**
**Hawthorne, NY 10532 (US)**

(72) Inventors:
• **GAO, Shen**
**Brampton, Ontario L6P 1B6 (CA)**
• **MOROS, Daniel**
**Larchmont, NY 10538 (US)**
• **MOLDENHAUER, Maxine**
**Acton, Ontario L7J 2L7 (CA)**

(74) Representative: **Chajmowicz, Marion et al**
**Becker & Associés**
**25 rue Louis le Grand**
**75002 Paris (FR)**

(56) References cited:
**EP-A- 0 468 232     EP-A- 0 479 005**
**WO-A-00/59467     FR-A- 2 713 931**
**US-A- 6 102 254**

**Description**

**BACKGROUND OF THE INVENTION**

The invention relates to a spill-resistant semi-solid pharmaceutical suspension for oral administration, comprising a suspension of an effective amount of water insoluble active ingredient such as ibuprofen in a pharmaceutically acceptable aqueous suspension-stabilizing vehicle.

Spill resistant pharmaceutical formulations for oral administration are described in U.S. Patent 6,071,523 issued to Mehta et al., and U.S. Patent 6,102,254 issued to Ross. There remains a need for suspension formulations, balancing the components of the formulation to achieve this goal while maintaining the characteristics of a spill resistant formulation.

References have been made to incorporating ibuprofen in a liquid suspension, as for example those disclosed in Mody et al., U.S. Patent 4,788,220, and Gowan, Jr., U.S. Patent 5,374,659. However, liquid suspensions are messy, require shaking before use, and present other problems. In addition, it is difficult to obtain a solution of ibuprofen active, and the solution formulation often has a bad taste. There remains a strong need for a palatable ibuprofen suspension.

EP 0479005 discloses spill resistant formulations comprising ibuprofen.

**SUMMARY OF THE INVENTION**

The invention relates to a semi-solid pharmaceutical suspension for oral administration, comprising a suspension of an effective amount of water insoluble active ingredient in a pharmaceutically acceptable aqueous suspension-stabilizing vehicle.

The invention provides pharmaceutical agents useful for systemic treatment by the oral route in a form which is convenient to administer to children, which is convenient for self administration of aging adults, as well as adults with motor problems, with improved taste.

The invention relates to a palatable ibuprofen oral suspension that is stable. This formulation is homogenous and does not require shaking before administration of the pharmaceutical product.

One embodiment of the invention is a palatable ibuprofen suspension as defined in claim 1. A suitable wetting agent (from 0.02% to 0.5%) can be included in the formulation to wet the ibuprofen particles. In one aspect of the invention, the ibuprofen spill resistant suspension comprises a carbomer-based gel in which the ibuprofen is dispersed rather than dissolved. To improve the sensory appeal, a high intensity sweetener such as sucralose has be added for additional sweetness. Poloxamer 188 can also be used as a wetting agent at a level of 0.05%. Glycerin can be adjusted to 39% and propylene glycol to 10% to equalize the density of the internal phase to the density of the ibuprofen. Sorbitol crystalline (about 5%) facilitates the dispersion of carbomer. The formulation is desirably 1.79% ibuprofen (equivalent to 100mg/5mL) with one of two flavors, cherry or berry. The levels of the carbomer can be adjusted to achieve optimal non-spill characteristics, e.g. 0.41 % for cherry flavor formula and 0.43% for berry flavor formula. The ibuprofen spill resistant suspension may include butylparaben, e.g. at a concentration of 0.018%.

The invention provides pharmaceutical agents useful for systemic treatment by oral administration in a composition which is provided in a device from which it is particularly easy to administer and convenient to measure single dosage units of the composition, and avoids the problems of liquid formulations, such as spillage.

The suspension may further comprise a crystal conditioning surfactant, e.g. from about 0.01 % to about 0.5% (w/w). The suspension may further comprise at least one organoleptic agent, such as food and drug color yellow number 6 and food and drug color red number 40, for example in a range from about 0.0025% to about 0.0075% (w/w). The crystal conditioning surfactant may be Poloxamer 188.

In any of the formulations of the invention the water-insoluble active ingredient is ibuprofen. The concentration may be about 1.79% (w/w). The active pharmaceutical ingredient may be in an immediate release formulation, a sustained release formulation, or a delayed release formulation.

The invention also relates to a pharmaceutical suspension comprising 1.79 % ibuprofen (w/w), 0.48% to 0.50% (w/w) Carbomer 934P, 0.08 % (w/w) sodium hydroxide, 0.05 % (w/w) Poloxamer 188, 10.0 % (w/w) propylene glycol, 39.0 % (w/w) glycerin, 5.0 % (w/w) sorbitol (crystalline), 0.40 % (w/w) sucralose liquid concentrate, 0.005 % (w/w) food and drug color yellow number 6, 0.20 % (w/w) masking agent, 0.83 % berry flavor, and 42 % purified water. The suspension may comprise up to about 0.18% (w/w) butylparaben or up to about up to about 0.04% (w/w) propylparaben.

The invention relates to a pharmaceutical suspension comprising an active pharmaceutical ingredient uniformly dispersed in an aqueous vehicle, the active ingredient remaining in suspension without agitation during the product shelf-life, wherein the density of the vehicle is approximately equal to the density of the active ingredient. The shelf life may be up to about six, twelve, eighteen, twenty-four months, thirty months, thirty-six months. The suspension has antimicrobial activity, pharmaceutically effective and satisfactory to meet applicable regulatory requirements as would be understood by a person of ordinary skill. The viscosity may be about 5,000 to about 20,000 mPa.s, about 5,000 to about 15,000 mPa.s, about 6,000 to about 17,000 mPa.s, or about 8,000 to about 11,000 mPa.s. In inventive pharmaceutical suspensions there is no crystalline growth during a heat-cool study for three days at 25°, 35°, or 45°C.

The pharmaceutical suspensions may comprise at least one additional component selected from the group consisting of excipients, surface active agents, dispersing agents, inert diluents, granulating agents, disintegrating agents, binding agents, lubricating agents, sweetening agents, flavoring agents, coloring agents, preservatives, oily vehicles, solvents, suspending agents, dispersing agents, wetting agents, emulsifying agents, demulcents, buffers, salts, fillers, antioxidants, antibiotics, antifungal agents and stabilizing agents.

In an inventive pharmaceutical suspension, ibuprofen at a concentration of about 1.79% (w/w) is suspended in a uniformly dispersed manner in an aqueous suspension without agitation during the product shelf-life and wherein the pharmaceutical suspension has the following properties: antimicrobial activity; a viscosity of between about 5,000 mPa.s to about 20,000 mPa.s or other viscosity ranges as described; a product shelf-life of up to about six months; no crystalline growth during a heat-cool study for three days at or 45°C; and an acceptable palatability. The Bingham behavior of the pharmaceutical suspension may have a yield value of 156 D/cm$^2$.

A suspension comprises (w/w) 0.5 to 5% ibuprofen, up to 1% organoleptic agents, from 0.4 to 0.5% Carbomer 934P, from 5 % to 10% Sorbitol (Crystalline), from about 10% to 20 % Propylene Glycol, from 33% to 41 % Glycerin, and up to 0.4 % Sucralose Liquid Concentrate. A particular suspension comprises about 42% water, about 39% glycerin, about 5% sorbitol and about 10% propylene glycol. Another suspension comprises about 52% water, about 24% sorbitol and about 20% propylene glycol. Another suspension comprises about 64% water, about 12 % glycerin and about 20% sorbitol. Another suspension comprises about 46% water and about 50 % glycerin. Another comprises about 29% water, about 47% glycerin and about 20% propylene glycol. Thus, the water may be in a range between about 29-64%, the glycerin may be in a range between about 0-50%, the sorbitol may be in a range between about 0-24%, and the propylene glycol may be in a range between about 0-20%. The density of the vehicle matches that of the active ingredient sufficiently to form a stable suspension.

The invention provides a pharmaceutical suspension for oral administration as defined above, comprising a suspension of an effective amount of particles of an active ingredient in a pharmaceutically acceptable aqueous suspension-stabilizing vehicle, the suspension having the following qualities:

> a homogeneity wherein the active ingredient is uniformly dispersed but not dissolved in the vehicle;
> a crystalline stability such that the active ingredient particles stay within a target particle size range during heat-cool studies;
> a suspension stability such that the active ingredient remains suspended during the product shelf-life without agitation;
> a Brookfield viscosity within the range of about 6,000 mPa.s to about 13,000 mPa.s at room temperature;
> an antimicrobial activity; and
> an acceptable palatability.

The active ingredient particles may be crystals that neither dissolve or grow substantially when the sample is heated e.g. to 45° C and cooled to room temperature repeatedly. The vehicle may have a density about equal to that of the active ingredient.

In suspensions of the invention, the composition can be squeezed into a spoon from a container with light manual pressure, to spread and level in a spoon bowl quickly enough for accurate measurement and to remain in the spoon bowl long enough to permit administration without spilling. In inventive suspensions, the composition spreads and levels in a spoon bowl within about 1-5 seconds at room temperature, and remains in the spoon bowl for, at least about 30 seconds on spoon inversion, about 30 seconds on spoon vibration, and about 1 second on spoon tilting. In exemplary formulations, the composition:

> (a) is non-Newtonian and a time independent fluid;
> (b) is pseudoplastic, and
> (c) exhibits Bingham behavior.

The compositions may have a yield value of about 156 D/cm$^2$.

## BRIEF DESCRIPTION OF THE DRAWINGS

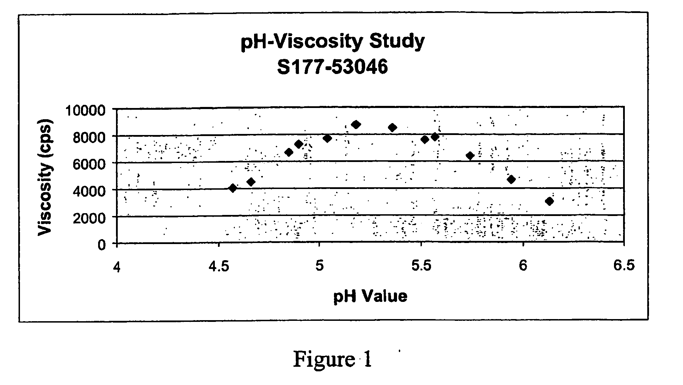

<u>Figure 1</u> demonstrates the pH - viscosity relationship (Carbomer 934P) for an ibuprofen spill resistant suspension. The viscosity of the formulation is dependent on the extent of carbomer neutralization. Sodium hydroxide is used to neutralize the carbomer with the preferred pH range being about 4.8 to about 5.5, or about 5 to about 5.4. The maximum viscosity is attained at around about pH 5.3 for the berry flavor formulas.

Figure 2 demonstrates the effect of the temperature in relation to the viscosity of an ibuprofen spill resistant suspension. An ibuprofen spill resistant suspension is heated from 15 °C to 45 °C and then cooled back to 15°C. The viscosity data is collected at each 5 °C interval to observe the effect of the temperature on the viscosity. The viscosity decreases as the temperature increases and the viscosity recovers completely as the temperature decreases.

Figure 3 demonstrates the flow viscosity relationship of an ibuprofen spill resistant suspension (23 °C, Spindle 21).

**DETAILED DESCRIPTION**

The invention relates to a pharmaceutical suspension for oral administration as defined in claim 1, comprising a suspension of an effective amount of particles of a water insoluble active ingredient in a pharmaceutically acceptable aqueous suspension-stabilizing vehicle. The inventive suspensions have some or all of the following qualities. First, the suspension may have a homogeneity wherein the active ingredient is uniformly dispersed but un-dissolved in the vehicle. It may have a crystalline stability such that the active ingredient does not exhibit excessive crystalline growth or dissolution, so that the particles stay within a target particle size range. Heat-cool studies can be conducted to check for crystal growth and active dissolution. For example, in an ibuprofen spill resistant suspension, after the sample is heated to 45° C and cooled to room temperature repeatedly, no dissolution of the ibuprofen is observed and there is no obvious crystal growth.

The suspension also may have suspension stability such that the active ingredient remains suspended indefinitely without agitation, that is without stirring or shaking. The elimination of the need to shake the suspension before administering is a significant advantage over the prior art, because the dosage is always uniform not requiring a minimal amount of shaking. This uniform suspension allows for consistent dosing and increased shelf life of the product, as the active ingredient remains uniform per dose administered, and does not fall out of solution. In Motrin ibuprofen suspension, for comparison, the particles settle. On the other hand, the Motrin suspension is a fluid and must be shaken to re-suspend. A semi-solid formulation of the invention can not be shaken easily, so the particles must remain suspended without shaking. Advantageously, there is no need to shake the inventive compositions. Suspension stability results from a reduced sedimentation rate.

The suspension may also have a Brookfield viscosity within the range of about 6,000 mPa.s to about 13,000 mPa.s at room temperature. Formulations exhibit desirable non-spill properties at a viscosity greater than about 6,000 mPa.s. The product spreads quickly at viscosity less than about 13,000 mPa.s. Thus spill resistance and spreading characteristics are desirable in this viscosity range. The viscosity of the ibuprofen spill resistant suspension is temperature sensitive between 15°C and 45°C. The viscosity of the formulation increases with decrease in temperature and decreases with increase in temperatures. However, these changes in the viscosity and correlated non-spill characteristics are reversible, so that the original formula viscosity is obtained when temperature returns to room temperature (~23°C; broadly 19°C to about 29°C).

The suspension may have a spill-resistant consistency permitting the composition to be squeezed into a spoon from a container with light manual pressure, to spread and level in a spoon bowl quickly enough for accurate measurement (typically in about 1-5 seconds at room temperature), and to remain in the spoon bowl long enough to permit administration without spilling particularly under difficult circumstances such as encountered with dispensing to children, or by the elderly. Spill-resistance refers to the product's ability to withstand a series of tests that were developed to evaluate the product's spill resistance, as seen in Example 3. For most formulations, spill resistance means the formulation does not spill from a teaspoon for a definite period, e.g. at least about 30 or 60 seconds on spoon inversion, about 30 or 60 seconds on spoon vibration, and about 10, 20, or 30 seconds on spoon tilting. Spill resistant properties correlate with viscosity but are not necessarily directly linked, so that a composition within the target viscosity range may lack spill resistance. The shaking, tilting and inversion tests are performed on an experimental platform as described in US Patent 6,071,523. Spill resistance is related to whether the formulation passes a flow test, ensuring that dispensing and dosing to a 5.0 mL teaspoon is easy and satisfactorily accurate.

The suspension may have a flow quality having a non-Newtonian, pseudoplastic and time independent fluidity wherein the viscosity of the non-solid gel decreases with increasing shear rate, in which the behavior is fully reversible, and is indicative of Bingham behavior. There is a relationship between flow and viscosity as seen in Example 5.

The ibuprofen spill resistant suspensions are non-Newtonian and time independent fluids. Non-Newtonian refers to a fluid whose behavior departs from that of an ideal Newtonian fluid. These fluids have different viscosities at different shear rates and fall under two groups: time independent and time dependent. In contrast, for a Newtonian fluid the rate of shear in the fluid under isothermal conditions is proportional to the corresponding stress at the point under consideration. Time independent fluids are those for which the rate of shear at any point in the fluid is some function of the shear stress at that point and depends on nothing else. These fluids have a constant viscosity value at a given shear rate. The viscosities do not change with time. (McGraw-Hill Encyclopedia of Science & Technology, 6th edition, 1987, Volume 12, pages 57-60).

The inventive suspensions may exhibit Bingham behavior with a yield value about 156.0 D/cm$^2$. Bingham plastics exhibit a yield stress, which is the stress that must be exceeded before flow starts. Thereafter the rate-of-shear curve is linear. There are other materials that also exhibit a yield stress, but the flow curve is thereafter not linear. These are usually called generalized Bingham plastics. A Bingham flow requires an initial stress, the yield value, before it starts to flow. Once the yield value is exceeded and flow begins a Bingham fluid may display Newtonian, pseudoplastic or dilatant flow characteristics. These fluids exhibit different behavior than thixotropic fluids.

The rheogram of these suspensions may be pseudoplastic. The viscosity of the gel decreases with increasing shear rate, and the behavior is fully reversible. Pseudoplastic fluid's ratio of shear stress to the rate of shear, which may be termed the apparent viscosity, falls progressively with shear rate. The decrease in viscosity with an increase in shear rate is also known as shear thinning. This phenomenon of shear thinning is characteristic of suspensions of asymmetric particles or solution of polymers such as cellulose derivatives. The viscosity of non-spill gel decreases with increasing the shear rate, e.g., increasing the spindle speed.

The inventive suspensions may have an antimicrobial activity satisfying microbial challenge requirements such as USP, either due to preservatives or a low water activity (about 0.752 to about 0.838). Propylparaben (up to about 0.04%) and Butylparaben (0.018% to about 0.18%) are suitable. These suspensions are alcohol-free to avoid complications from using alcohol and have palatability such that the suspension has an acceptable taste and good mouthfeel.

The inventive suspension comprises an active ingredient and a vehicle. The active ingredient is pharmaceutically active, e.g. ibuprofen, is insoluble in the vehicle in that the ibuprofen is not dissolved in the non-spill gel base, and is suspendable. Suspensions are defined as a class of materials in which one phase, as solid, is dispersed in a second phase, generally a liquid. Here, the ibuprofen is dispersed homogeneously in the base and has an equal density to the vehicle.

The inventive suspension also comprises a vehicle that is pharmaceutically acceptable, aqueous, and suspension-stabilizing, comprising a thickener component and a carrier component, and may include organoleptic components.

The thickener provides the necessary viscosity, spill-resistant properties such as pseudoplasticity, and to suspend the active agent. Carbomers (Merck Index 12th ed., no. 1878) can be used as thickeners in semisolid pharmaceutical formulations (see Mehta et al., US patent 6,071,523). Carbomer 934P (Carbopol® 974P) is a suitable thickener or gelling agent Suitable concentrations range preferably from about 0.40 to about 0.48 %, w/w. Its rheology supports a high yield value. (Handbook of Pharmaceutical Excipients Third Ed., A.H. Kibbe (Ed.), Pharmaceutical Press, London, UK., 2000, Pg. 442, 79, 53 ("Handbook of Pharm. Excipients")) Carbomers are slightly acidic and must be neutralized e.g. with sodium hydroxide (as needed to neutralize the carbomer up to about 0.08 % in particular formulations) with a pH range being 4.8 to 5.6, providing the maximum viscosity plateau.

The formulation may require crystal conditioning surfactant (a wetting agent) that prevents the active agent particles from floating. An example of a wetting agent is the non-ionic surfactant Poloxamer 188, known as Pluronic F68 (T.D.S.-214 Carbopol B.F.Goodrich Company The Merck Index 12th Ed., Merck&Co. Inc. 1996, p. 839), which at a concentration of 0.05% (w/w) can completely wet ibuprofen particles. (Pharmaceutical Dosage Forms: Disperse System, Volume 1, Marcel Dekker, Inc., New York and Basel., 1988, Pg. 181 ("Pharm. Dosage Forms: Disperse System"). Other satisfactory surfactants may be used, for example those known in the art such as other poloxamers. Suitable concentrations of surfactants range from about 0.01 % to about 0.5% depending on the content of solids intended for suspension. Concentrations less than about 0.05% can result in incomplete wetting. Surfactant concentrations greater than about 0.5% may solubilize ultrafine particles and eventually lead to changes in particle size distribution and crystal growth. (Pharm. Dosage Forms: Disperse System).

The carrier component primarily serves as the external phase of the suspension matching the density of the active agent, and as the liquid providing necessary flow characteristics, and also contributes other properties to the suspension. The carrier component comprises propylene glycol from 10% to 20%. Propylene glycol is widely used as a solvent, extractant, and preservative in a variety of pharmaceutical formulations.

The carrier also comprises glycerin from 33% to 39%. A suitable glycerin concentration of the formula is 39% w/w with 10% propylene glycol to equalize the density of the internal phase to the density of the ibuprofen, as seen in Example 9.

Purified water makes up the bulk of the carrier component . Water concentration can be less than about 50% w/w or even less than about 43 % in ibuprofen formulations.

The carrier component of the suspension also comprises sorbitol at a level of 5-10% w/w to facilitate the carbomer dispersion and provide sweetness.

The suspension also comprises organoleptic components, which impart desirable sensory characteristics to the suspension, including taste, color, and smell. The organoleptic component may comprise a high intensity sweetener that improves sensory appeal such as sucralose liquid concentrate up to about 0.40%, and more specifically from about 0.005 to about .020%.

These components may also include coloring agents that provide desired shades consistent with berry or cherry flavor products such as FD&C Yellow #6 or FD&C Red #40 from about 0.0025 % to about 0.0075%. Flavoring agents such as about 0.15% cherry flavor or a concentration of about 0.83% berry flavor, and taste masking agents may be included to obscure the bitter flavor of active agents such as ibuprofen.

A formulation of the invention may include about 1.79 % Ibuprofen, about 0.48% to 0.50% Carbomer 934P (Carbopol® 974P), about 0.08 % Sodium Hydroxide, about 0.05 % Poloxamer 188, about 10.0 % Propylene Glycol, about 39.0 % Glycerin, about 5.0 % Sorbitol (Crystalline), about 0.40 % Sucralose Liquid Concentrate, about 0.005 % FD&C Yellow #6, about 0.20 % Masking Agent, about 0.83 % Berry Flavor, and about 42 % Purified Water, optionally with about .018% Butylparaben.

Where the term "pharmaceutical" is used herein, it should be understood to include prescription, over the counter, GRAS (generally recognized as safe), nutraceutical, and other products whether subject to approval by a drug regulatory agency or not.

Any conventional technique may be used for the preparation of pharmaceutical formulations according to the invention. The active ingredient may be contained in a formulation that provides quick release, sustained release or delayed release after administration to the patient.

Pharmaceutical compositions that are useful in the methods of the invention may be prepared, packaged, or sold in formulations suitable for oral, parenteral and topical administration. Other contemplated formulations include nanoparticles, liposomal preparations, resealed erythrocytes containing the active ingredient, and immunologically-based formulations.

The formulations of the pharmaceutical compositions described herein may be prepared by any method known or hereafter developed. In general, preparation includes bringing the active ingredient into association with a carrier or one or more other additional components, and then, if necessary or desirable, shaping or packaging the product into a desired single- or multi-dose unit.

As used herein, "additional components" include, but are not limited to, one or more of the following: excipients; surface active agents; dispersing agents; inert diluents; granulating and disintegrating agents; binding agents; lubricating agents; sweetening agents; flavoring agents; coloring agents; preservatives; physiologically degradable compositions such as gelatin; aqueous vehicles and solvents; oily vehicles and solvents; suspending agents; dispersing or wetting agents; emulsifying agents, demulcents; buffers; salts; thickening agents; fillers; emulsifying agents; antioxidants; antibiotics; antifungal agents; stabilizing agents; pharmaceutically acceptable polymeric or hydrophobic materials as well as other components.

Although the descriptions of pharmaceutical compositions provided herein are principally directed to pharmaceutical compositions which are suitable for administration to humans, it will be understood by the skilled artisan, based on this disclosure, that such compositions are generally suitable for administration to any mammal. Preparation of compositions suitable for administration to various animals is well understood, and the ordinarily skilled veterinary pharmacologist can design and perform such modifications with routine experimentation based on pharmaceutical compositions for administration to humans.

A pharmaceutical composition of the invention may be prepared, packaged, or sold in bulk, as a single unit dose, or as a plurality of single unit doses. As used herein, a "unit dose" is a discrete amount of the pharmaceutical composition comprising a predetermined amount of the active ingredient. The amount of the active ingredient in each unit dose is generally equal to the total amount of the active ingredient which would be administered or a convenient fraction of a total dosage amount such as, for example, one-half or one-third of such a dosage.

Suspensions, in which the active ingredient is dispersed in an aqueous or oily vehicle, and liquid solutions, in which the active ingredient is dissolved in an aqueous or oily vehicle, may be prepared using conventional methods or methods to be developed. Liquid suspension of the active ingredient may be in an aqueous or oily vehicle and may further include one or more additional components such as, for example, suspending agents, dispersing or wetting agents, emulsifying agents, demulcents, preservatives, buffers, salts, flavorings, coloring agents, and sweetening agents. Oily suspensions may further comprise a thickening agent. Liquid solutions of the active ingredient may be in an aqueous or oily vehicle and may further include one or more additional components such as, for example, preservatives, buffers, salts, flavorings, coloring agents, and sweetening agents.

The term "spill resistant formulation" refers here to a product which, as sold, has viscosity in a certain range (e.g. 5,000 to 20,000 mPa.s), is a semi-solid, is easy to administer accurately, has spill-resistant consistency, is storage stable, and has mutually compatible ingredients, as described in Mehta et al., US 6,071,523. Viscosity can be measured using a Brookfield Viscometer with a 'T-C' spindle at 20 RPM and 20-25 degrees C, or equivalent. Viscosity decreases slightly with increasing temperature.

Semi-solid character in this context generally indicates a formulation that has a viscometric yield value determined as a relative value, e.g. using a Brookfield Viscometer to measure a shear vs. stress curve. Ease of administration is intended to mean (a) extrudability under light manual pressure from a squeezable container or a proxy (e.g. a syringe with a 5 mm orifice), and (b) spreadability in a spoon bowl measured by extruding the formulation into a spoon bowl and determining whether the material levels or spreads to the edges of the spoon bowl. Spreadability also contributes to accuracy of measurement.

A spill-resistant formulation according to the invention begins to spill from a spoon bowl during test periods of vibrations, inversion, and tilting, but slowly enough to conform with practical time limits between dispensing and ingesting, and quickly enough to enable the product to be readily consumed from a spoon bowl by a patient.

Mutual compatibility of the components means that they do not separate in preparation and storage for the equivalent of two years at room temperature (as indicated by three months accelerated stability testing at 40° centigrade and 75% relative humidity). Storage stability means that the materials do not lose their desirable properties during storage for the same period. Preferred compositions do not exhibit a drop in viscosity of more than 50% or an increase in viscosity of more than 100% during that period.

The inventive formulations have attractive appearance, suitable texture and organoleptic (taste and mouth-feel) properties. The components are mutually compatible in that they do not interfere with the bioactivity of the pharmaceutical agent or physical properties of the vehicle, and the components do not separate and retain their properties.

The present invention can be understood using a general equation for Stokes' law, as follows (Pharmaceutical Dosage Forms: Disperse System, Volume 2, Marcel Dekker, Inc., New York and Basel., 1996, Pg. 152 ("Pharm. Dosage Forms Vol. 2"):

$$V = d^2 \text{ x } (\rho_S - \rho_L)g \, / \, 18\eta$$

Wherein

V represents settling velocity,

d represents Stokes' diameter,

$\rho_S$ represents density of solid,

$\rho_L$ represents density of liquid,

$g$ represents acceleration due to gravity, and

$\eta$ represents viscosity of liquid.

According to the Stokes' law, reducing the sedimentation rate can be achieved by the following methods: (1) decreasing the particle size of the suspended phase, (2) minimizing the difference of the density between the suspended phase and the external phase (liquid phase), and (3) increasing the viscosity of the external phase. Most suspension development focuses on the particle size rather than equalizing the density between the suspended phase and the external phase. Suspensions of the present invention have a unique combination of ingredients that provide an external phase with a density about equal to the active.

An example of a preservative-free suspension comprises about 42 % Purified Water, about 0.005 % FD&C Yellow #6, about 0.05 % Poloxamer 188, about 0.08 % Sodium Hydroxide, about 0.48% Carbomer 934P (Carbopol® 974P), about 5.0 % Sorbitol (Crystalline), about 10.0 % Propylene Glycol, about 1.79 % Ibuprofen, about 39.0 % Glycerin, about 0.40 % Sucralose Liquid Concentrate, about 0.20 % Masking Agent, and about 0.83 % Berry Flavor.

An example of a suspension with a preservative may comprise about 43 % Purified Water, about 0.005 % FD&C Yellow #6, about 0.05 % Poloxamer 188, about 0.08 % Sodium Hydroxide, about 0.5% Carbomer 934P (Carbopol® 974P), about 5.0 % Sorbitol (Crystalline), about 10.0 % Propylene Glycol, about .018% Butylparaben, about 1.79 % Ibuprofen, about 39.0 % Glycerin, about 0.40 % Sucralose Liquid Concentrate, about 0.20 % Masking Agent, and about 0.83 % Berry Flavor.

A suitable glycerin concentration of the formula is 39% w/w, and 10% of propylene glycol. All percentages here are given as weight-weight as the density of the formulations differs from that of water.

It is an object of the invention to prevent the active from floating in the suspension. A suitable wetting agent as discussed above with a pleasant smell and taste is therefore required. An example of a good wetting agent is Poloxamer 188, which at a concentration of 0.05% of the solution could completely wet the ibuprofen particles.

In order to optimize the viscosity of the gel, the carbomer vs. viscosity relationship can be evaluated. A required carbomer level of about 0.3% can impart a viscosity of approximately 10,000 mPa.s which would provide desirable non-spill properties of the formula. However, other excipients such as flavor concentrate can also affect the viscosity of the formula.

In yet another embodiment, the suspension may further contain a preservative, such as butylparaben in amounts of about 0.006 to about 0.05%, and more specifically about 0.018%.

In contrast to prior formulations, e.g. Mody et al., U.S. Patent 4,788,220, and Gowan, Jr., U.S. Patent 5,374,659, the spill resistant pharmaceutical suspension does not contain gum-like suspending agents such as xanthan gum. Such thickeners are incompatible with the desired characteristics of the inventive spill-resistant formulations.

The pharmaceutical compositions of the invention comprise a pharmaceutical agent in an effective amount for systemic treatment by oral administration in admixture with a pharmaceutically acceptable vehicle comprising a thickening agent in a amount which provides a semisolid, such as a gel or a paste suspension. The semisolid has a Brookfield viscosity in a range of at least about 5,000, 6,000, 7,000, 8,000 mPa.s, and less than about 11,000, 12,000, 13,000 or 15,000. Thus, desirable ranges include about 5,000-15,000 mPa.s, 5,000-20,000 mPa.s, 6,000-17,000, or about 8,000 to about 11,000 mPa.s. In the present application, viscosity refers to Brookfield viscosity, measured at about 25°C, and at a spindle speed of 10 rpm, unless otherwise noted, which measures viscosity of pseudoplastic materials.

In general, the viscosity of the compositions of the invention can be varied by the choice and amount of thickening agent and other components to a consistency which permits the composition to be readily squeezed and flow through a relatively narrow orifice, i.e. of the order of about 1 to 10 mm in diameter.

Systemic treatment relates to treatment which affects the body as a whole, as compared to topical treatment, which affects only that part of the body to which it is applied, i.e. skin, teeth or particular mucous membrane, such as the lining of the stomach.

The semisolid compositions of the invention have a liquid base, which is a palatable pharmaceutically acceptable solvent, which may dissolve or suspend the active pharmaceutical agent. Solvents include water, propylene glycol, glycerin, polyethylene glycol and mixtures thereof.

The inventive pharmaceutical suspension for oral administration is adapted to be used in conjunction with a device or package that makes it particularly easy to measure single dosage units of a pharmaceutical agent useful for systemic treatment and convenient to administer them orally in a semi-solid composition. These devices would particularly be suitable for administration to children and for self-administration by aging adults, and adults with motor problems. They are resistant to tampering by young children or individuals with limited mental capacity due to a childproof closure.

For example, bottles of different resin types, such as polyethylene (PE) and low density polyethylene (LDPE), and different shapes can be used to deliver various spill resistant pharmaceutical compositions. The squeezability of a 4-oz custom made bottle made using polyethylene terephthalate (PETG) material is satisfactory and controlled delivery of the spill resistant pharmaceutical compositions. PETG has a number average molecular weight of approximately 26,000. Various plugs of different architecture can also be used; e.g. plugs of LDPE are acceptable, including Huntsman PE 2030 or another polymer with similar characteristics. The inventive formulation can be used with a variety of other packaging components.

The following examples further illustrate the invention, but should not be construed as limiting the invention in any manner.

**EXAMPLE 1**

Ibuprofen spill-resistant suspension BERRY FLAVOR SUSPENSION IBUPROFEN USP, 1.79% (Equivalent to 100mg/5mL)

50 Kg Batch

**Table 1**

| INGREDIENTS | DEC% | QUANTITY grams |
|---|---|---|
| Ibuprofen | 1.79 | 895.0 |
| Purified Water | 42.0 | 21322.5 |
| Glycerin | 39.0 | 19,500 |
| Sorbitol (Crystalline) | 5.0 | 2,500.0 |
| Propylene Glycol | 10.0 | 5,000 |
| Carbomer 934P (Carbopol® 974P) | 0.48 | 240.0 |
| Poloxamer 188 | 0.05 | 25.0 |
| FD&C Yellow #6 | 0.005 | 2.5 |
| Sodium Hydroxide | 0.08 | 40.0 |
| Sucralose Liquid Concentrate | 0.40 | 200.0 |
| Masking Agent #141.18074 | 0.20 | 100.0 |
| Berry Flavor | 0.83 | 415.0 |

Deviation allowed for 50 Kg is from about 47.5 Kg to about 50.5 Kg; equivalent to about 95% to about 101%.

REPARATION OF SUSPENSION

**Step 1**

(a) 21,322.5 grams Purified Water, 2.5 grams FD&C Yellow #6 and 25 grams Poloxamer 188

(b) Add the purified water into a stainless steel pot and retain approximately 200 grams for rinsing in step # 7. Add and dissolve the FD&C Yellow #6 and poloxamer 188 by stirring manually with spatula until dissolved completely.

**Step 2**

(a) 360 grams Purified Water, 40 grams Sodium Hydroxide

(b) Add the purified water into a stainless steel pot. Add and dissolve the sodium hydroxide manually using a spatula to form a clear solution. Cover the solution and retain for using in step #8.

**Step 3**

(a) 20,000 grams Purified Water, 240 grams Carbomer 934P

(b) Add the purified water into the preparation kettle. Install the mixer (Lightnin, Rochester NY) and adjust the mixer speed and position to yield a vortex and maintain the vortex. Slowly add the carbomer to the preparation kettle. Mix for a minimum of 20 minutes or until a lump free dispersion has formed. Immerse a spatula into the slurry. When a smooth slurry is formed with no carbomer lumps remaining on the spatula, the carbomer is completely dispersed.

**Step 4**

(a) 2,500 grams Sorbitol Crystalline

(b) Add the sorbitol crystalline to the carbomer dispersion. Maintain the speed of the mixer from step #3 until all the sorbitol is dissolved. Immerse a spatula into the slurry. When a smooth slurry is formed with no sorbitol lumps remaining on the spatula, the sorbitol crystalline is completely dissolved.

(c) Adjust the agitation speed to achieve movement of the bulk without a vortex. Continue mixing until the beginning of step #6.

**Step 5**

(a) 5,000 grams Propylene Glycol, 895 grams Ibuprofen

(b) Add propylene glycol into a stainless steel pot and remove about 1,000 grams for rinsing in step #6. Add ibuprofen to the propylene glycol and mix manually until a lump free dispersion has formed. Immerse a spatula into the slurry. When a smooth slurry is formed with no ibuprofen lumps remaining on the spatula, the ibuprofen is completely dispersed.

**Step 6**

(a) 19, 500 grams Glycerin, 200 grams Sucralose Liquid Concentrate

(b) Add glycerin and sucralose liquid concentrate into the Brogli Mixing Vessel. Set the agitator speed to 40 ± 15 rpm. Add ibuprofen dispersion from step #5 into the Brogli Mixing Vessel. Rinse the container with the propylene glycol retained from step #5 and add the rinse into the Brogli Mixing Vessel.

(c) Add carbomer dispersion from step #6 into the Brogli Mixing Vessel. Continue to mix for 10 ± 2 minutes

**Step 7**

(a) 100 grams Masking Agent, 415 grams Artificial Berry Flavor

(b) Set the agitator speed at 45 ± 10 rpm. Add the berry flavour, the masking agent, F.D.C Yellow #4 and poloxamer 188 solution from step #1 to the Brogli Mixing Vessel. Rinse with the water retained from step #1 and add the rinse to the Brogli Mixing Vessel. Continue mixing for 10 ± 5 minutes.

**Step 8**

(a) Adjust the agitator speed to 40 ± 10 rpm. Calculate as (carbomer grams X 280) /24 grams = weight ofNaOH. Add sodium hydroxide solution from step #2. Mix for 10 ± 5 minutes.

(b) Check the pH of the gel undiluted. Target: 5.3. Limit: 5.0 to 5.6.

(c) If necessary, adjust the pH of the gel with 10 grams increments of sodium hydroxide solution prepared from step #2. Mix the batch for 10 ± 5 minutes after each addition of sodium hydroxide solution. Record the addition weight of sodium hydroxide solution used to adjust the pH.

The manufacturing process may be better understood with Figure 1.

## EXAMPLE 2

Ibuprofen spill-resistant suspension BERRY FLAVOUR SUSPENSION (with Preservative)

IBUPROFEN USP, 1.79% (Equivalent to 100mg/ 5mL)
Batch Size: 50.0 kg

**Table 2**

| INGREDIENTS | % | QUANTITY-G |
|---|---|---|
| Ibuprofen | 1.79 | 895.0 |
| Purified Water | 43.0 | 21313.5 |
| Glycerin | 39.0 | 19,500 |
| Sorbitol (Crystalline) | 5.0 | 2,500.0 |
| Propylene Glycol | 10.0 | 5,000 |

(continued)

| INGREDIENTS | % | QUANTITY-G |
|---|---|---|
| Carbomer 934P (Carbopol® 974P) | 0.50 | 250.0 |
| Poloxamer 188 | 0.05 | 25.0 |
| FD&C Yellow #6 | 0.005 | 2.5 |
| Sodium Hydroxide | 0.08 | 40.0 |
| Sucralose Liquid Concentrate | 0.40 | 200.0 |
| Masking Agent #141.18074 | 0.20 | 100.0 |
| Berry Flavor | 0.83 | 415.0 |
| Butylparaben | 0.018 | 9.0 |

## EXAMPLE 3

### Non-Spill Properties and Tests

"Non-spill properties" refers to the product's ability to pass a series of tests that are developed to evaluate the product's spill resistance. The non-spill properties correlate with viscosity at a given temperature. The shaking test, tilting test and inversion test are used to determine resistance to spilling and the fourth, the flow test, is intended to ensure the product viscosity is such that dispensing and dosing to a 5.0 mL spoon is satisfactory (as disclosed in US Patent No. 6,071,523).

Non-spill properties for ibuprofen spill resistant suspensions and prior art formulations were compared in Table 3.

**Table 3: Non-spill test results for various batches**

| Sample | Lot No. | Viscosity[1] mPa.s | Non-spill tests as per protocol (Spill Resistant Time) | | | |
|---|---|---|---|---|---|---|
| | | | Spreading[2] | Inversion Sec | Vibration Sec. | Tilting sec. |
| Ibuprofen spill resistant suspension Cherry Flavor | S177-52679 | 8810 | 2 | > 60 | > 60 | > 60 |
| Ibuprofen spill resistant suspension Cherry Flavor | S177-52683 | 8150 | 2 | > 60 | > 60 | > 60 |
| Advil | 992186 | 1810 | 1 | Immediately | Immediately | Immediately |
| Motrin | DMF014 | 550 | 1 | Immediately | Immediately | Immediately |
| [1] VIS-02, spindle C, 20 rpm, room temperature 2 Spreading was measured on a scale where 1 is more fluid, 2 is a Nonspil semi solid, 3 is a stiffer semi solid and 4 is very stiff, (the product did not spread). | | | | | | |

The ibuprofen spill resistant suspension's non-spill properties for various viscosity measurements are set forth in Table 4. The ibuprofen spill resistant suspension's non-spill properties for various viscosity measurements are set forth in Table 4. The non-spill properties depend on viscosity at a specific temperature.

At 23°C, the product exhibits desirable non-spill properties at a viscosity of greater than about 6,000 mPa.s. However, at this temperature, the product can not spread well if the viscosity is more than about 13,000 mPa.s. Therefore, ibuprofen spill resistant suspension has a good non-spill and spreading characteristics at a viscosity range from about 6,000 mPa.s to about 13,000 mPa.s.

**Table 4: Relationship of Viscosity and Non-Spill Properties**

| Viscosity (cps) | pH | Temp. (°C) | Non-spill tests as per protocol (Spill Resistant Time) | | | |
|---|---|---|---|---|---|---|
| | | | Inversion sec | Vibration sec. | Tilting sec. | Spreading[1] |
| 4050 | 4.57 | 23 | < 1 | 14 | 1 | 1 |
| 4490 | 4.66 | 23 | 4 | > 60 | 1 | 1 |
| 6670 | 4.85 | 23 | > 60 | > 60 | 7 | 1.5 |
| 7270 | 4.90 | 23 | > 60 | > 60 | 13 | 1.5 |
| 7700 | 5.04 | 23 | > 60 | > 64 | > 60 | 1.5 |
| 8710 | 5.18 | 23 | > 60 | > 60 | > 60 | 2 |
| 9560 | 5.18 | 20 | > 60 | > 60 | > 60 | 2 |
| 10460 | 5.18 | 18 | > 60 | > 60 | > 60 | 2 |
| 11260 | 5.18 | 16 | > 60 | > 60 | > 60 | 2 |
| 12310 | 5.18 | 14 | > 60 | > 60 | > 60 | 2 |
| 13140 | 5.18 | 12 | > 60 | > 60 | > 60 | 2.5 |
| 14460 | 5.18 | 10 | > 60 | > 60 | > 60 | 3 |
| spreading was measured on a scale where 1 is more fluid, 2 is Nonspil semi solid, 3 is a stiffer semi solid and 4 is very stiff (that is the product did not spread). | | | | | | |

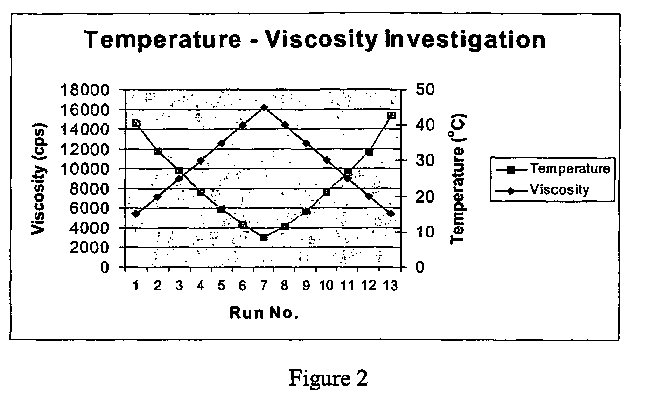

The viscosity of the formulation was also dependent on the extent of carbomer neutralization. The end point pH range of 4.9 to 5.8 was determined at the initial development stage. The pH vs. viscosity plot showed that the maximum viscosity is attained at around pH 5.3 for berry flavor formulas. See Figure 2.

Sodium hydroxide was used to neutralize the carbomer with the preferred pH range being 4.8 to 5.5. The maximum viscosity plateau is obtained at this pH range. The relationship with the pH value, viscosity, and the non-spill properties can be seen in Table 5.

**Table 5: Relationship Among pH, Viscosity, and Non-Spill Properties**

| pH Value | Viscosity (mPa.s) | Non Spill Properties | | | |
|---|---|---|---|---|---|
| | | Inversion (Sec) | Tilting (Sec) | Shaking (Sec) | Spreading[1] |
| 4.57 | 4050 | < 1 | 14 | 1 | 1 |
| 4.66 | 4490 | 4 | > 60 | 1 | 1 |
| 4.85 | 6670 | > 60 | > 60 | 7 | 1.5 |
| 4.9 | 7270 | > 60 | > 60 | 13 | 1.5 |
| 5.04 | 7700 | > 60 | > 60 | > 60 | 1.5 |
| 5.18 | 8710 | > 60 | > 60 | > 60 | 2 |
| 5.36 | 8480 | > 60 | > 60 | > 60 | 1.5 |
| 5.52 | 7610 | 55 | > 60 | > 60 | 1.5 |
| 5.57 | 7820 | 54 | > 60 | > 60 | 1.5 |
| 5.74 | 6410 | 19 | > 60 | > 60 | 1.5 |
| 5.94 | 4640 | 1 | 37 | 2 | 1 |

(continued)

| pH Value | Viscosity (mPa.s) | Non Spill Properties | | | |
|---|---|---|---|---|---|
| | | Inversion (Sec) | Tilting (Sec) | Shaking (Sec) | Spreading[1] |
| 6.13 | 3020 | < 1 | 2 | 1 | 1 |
| [1] Spreading was measured on a scale where 1 is more fluid, 2 is Nonspil semi solid, 3 is a stiffer semis solid and 4 is very stiff (that is the product did not spread). | | | | | |

The formula maintained non-spill properties at the pH range of about 4.8 - 5.5 (viscosity not less than 6,000 mPa.s). The flow is not greater than 2 within this range. Therefore, the pH value for this formulation should be within the range of about 4.9 to about 5.5.

## EXAMPLE 4

### Temperature - Viscosity Relationship

The viscosity of ibuprofen spill resistant suspension varied with the temperature. Ibuprofen spill resistant suspension was adjusted to different temperatures using a water bath and the viscosity data was collected under each condition.

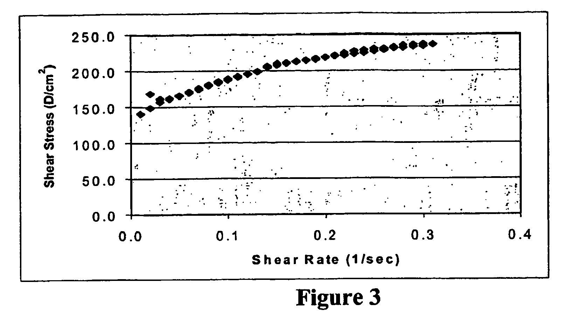

In this example, the viscosity of the ibuprofen spill resistant suspension changed as the temperature changed. The ibuprofen spill resistant suspension was heated from 15°C to 45°C and then cooled back to 15°C. The viscosity data was collected at each 5°C interval to observe the effect of the temperature on the viscosity. Figure 3 shows the effect of temperature on the viscosity of an ibuprofen spill resistant suspension. The results indicate that the viscosity decreased as the temperature increased, and the viscosity recovered completely as the temperature decreased.

## EXAMPLE 5

### Flow and Viscosity Profile

Ibuprofen spill resistant suspension was subjected to a viscosity behavior study. A Model VISO2 Rheometer (Brookfield, Middleboro, MA U.S.A) equipped with small sample adapter was used for this purpose. The start set speed of the spindle was 0.01 rpm. The speed was increased 0.01 rpm every 15 seconds until the speed reached 0.31 rpm. Then the speed was decreased 0.01 rpm every 15 seconds until the speed went back to 0.01 rpm. The viscosity and shear stress were measured every time before the speed changed. The flow curve rheogram was generated. Figure 4 shows the flow and viscosity profile of an ibuprofen spill resistant suspension (23°C, Spindle 21).

These examples demonstrate that the ibuprofen spill resistant suspension is non-Newtonian and time independent fluid and pseudoplastic. The viscosity of the gel decreased with increasing shear rate, and the behavior was fully reversible. From the rheogram, the Bingham yield value was calculated to be 156.0 $D/cm^2$.

## EXAMPLE 6

### Ibuprofen Heat - Cool Studies

Heat - cool studies were conducted to check for crystal growth and active dissolution in the ibuprofen spill resistant suspension. The studies were performed at different temperature as in Tables 6-8.

Table 6: Summary of the results of the ibuprofen spill resistant suspension heat-cool study

| Lot No. "A" | Microscopic Appearance after (days at 30 °C/days at 6 °C) | | | | | |
|---|---|---|---|---|---|---|
| Days | 4/2 | 2/3 | 2/2 | 3/2 | 2/3 | 2/2 |
| Crystal Growth | None | None | None | None | None | None |
| Viscosity (mPa.s) [1] | 8250 | 8390 | 7940 | 8010 | 7820 | 7840 |
| [1] VIS-02, Spindle C at 21-23 °C, 20rpm The initial viscosity is 8600 mPa.s | | | | | | |

**Table 7: Summary of the results of the ibuprofen spill resistant suspension heat-cool study No. 2**

| Lot No. "A" | Microscopic Appearance after (days at 40 °C/days at 6 °C) | | | | | |
|---|---|---|---|---|---|---|
| Days | 4/2 | 2/3 | 2/2 | 3/2 | 2/3 | 2/2 |
| Crystal Growth | None | None | None | None | None | None |
| Viscosity (mPa.s)[1] | 8540 | 7890 | 7740 | 7740 | 7870 | 7750 |
| [1] VIS-02, Spindle C at 21-23 °C, 20rpm. The initial viscosity is 8600 mPa.s. | | | | | | |

**Table 8: Summary of the results of the ibuprofen spill resistant suspension heat-cool study No 3**

| Lot No. "A" | Microscopic Appearance after (days at 45 °C/days at room temperature) | | | | |
|---|---|---|---|---|---|
| Days | 3/1 | 1/1 | 1/3 | 1/1 | 1/1 |
| Crystal Growth | None | None | None | None | None |
| Active Dissolution | None | None | None | None | None |

After the sample was stored at 30 C, 40 C or 45°C for 2 to 3 days and cooled to room temperature, no dissolution of the ibuprofen was observed and there was no obvious crystal growth found in the ibuprofen spill resistant suspension during all heat-cool cycle studies.

**Ibuprofen Suspension Heat - Cool Study**

A suspension was subjected to a series of heat (45 C) for 1 day and cooled (room temperature, 23 C) for 1 day studies to determine whether the active dissolved and whether crystal growth occurred. The results are given in Table 9.

**Table 9- Heat/Cool 1 Day Study**

| Sorbitol %w/w | Glycerin %w/w | Water %w/w | Calculated Water Activity | Actual Water Activity* | Cycle 1 | Cycle 2 | Cycle 3 |
|---|---|---|---|---|---|---|---|
| 5 | 39 | 42 | 0.838 | 0.756 | No Obvious Crystal Growth | No Obvious Crystal Growth | No Obvious Crystal Growth |
| * Data was obtained from water activity equipment that was not calibrated. | | | | | | | |

Results showed that the ibuprofen crystal did not dissolve under the conditions of the study. (that is, no crystal growth was observed after 5 cycles of 45 C/23 C).

**Ibuprofen Suspension Freeze-Thaw Study**

Ibuprofen batches were stored at 6 C for 1 day freeze/thaw studies to determine whether the crystallization occurred and whether crystal growth occurs. Results showed that no crystal was observed after 5 cycles of study (data not shown)

**EXAMPLE 7**

**In Vitro Release/Dissolution**

Three products, Motrin, Advil and Taro NonSpil gel, were used to test for the dissolution limits. Amount of dissolved active (% label claim) was adopted directly from the Ibuprofen Suspension USP Dissolution procedure for ibuprofen oral suspension. Dissolution testing results measure the amount of Ibuprofen dissolved in 60 minutes in a dissolution apparatus using a spindle at 50 rpm (revolutions per minute) at 37°C. A dissolution apparatus (Distek or equivalent), equipped with 6 to 8 vessels immersed in a water bath maintained at 37±0.5°C and fitted with individual spindles for each vessel was employed fore the testing. Samples taken by syringe at regular intervals from each vessel and active measured by reverse-phase HPLC, using Phenomenex Luna C18 column with the mobile phase containing methanol/acetonitrile/phosphate buffer at pH 2.3 (120/360/520), eluting at 1.5 mL/min for 25 minutes, then 3.0 cumin for 12 minutes for the column wash, and detection by UV absorption at 220 nm. Ibuprofen samples were prepared for analysis by dissolving about 5.5 grams of product in the sample solvent and its subsequent dilution in the sample solvent to desirable concentration. The typical retention time of preservative (butylparaben)is 6.76 minutes, ibuprofen 15.20 minutes and identified impurity (4-Isobutylacetophenone) 17.22 minutes.

**Table 10: Dissolution Testing Using HPLC Quantitation using the USP24 method**

| Sample | 3 min | 6 min | 9 min | 12 min | 15 min | 30 min | 45 min | 60 min |
|---|---|---|---|---|---|---|---|---|
| Motrin-1 | 92.6 | 94.0 | 93.3 | 92.6 | 92.1 | 91.2 | 88.9 | 88.9 |
| Motrin-2 | 93.2 | 94.0 | 93.4 | 92.6 | 92.3 | 91.1 | 89.3 | 89.1 |
| Advil-1 | 35.4 | 44.8 | 49.9 | 56.3 | 63.2 | 91.7 | 92.9 | 92.7 |
| Advil-2 | 39.5 | 48.1 | 52.9 | 61.5 | 67.6 | 91.9 | 92.5 | 92.2 |
| Taro-1 | 50.6 | 66.4 | 75.0 | 83.0 | 86.8 | 91.5 | 90.2 | 89.1 |
| Taro-2 | 50.1 | 66.3 | 76.8 | 82.7 | 86.9 | 92.1 | 90.6 | 89.4 |

## EXAMPLE 8

A method of determining an appropriate amount of vehicle components to achieve a suspension wherein the active neither sinks nor floats involves titrating the concentration of the component as follows. In order to match the density, an experiment was conducted with varying the glycerin amount. Ten ibuprofen suspension batches were compounded with different levels of glycerin. One non-neutralized sample from each of the above ten batches was centrifuged at 2500 rpm for one hour at room temperature. The results are shown in Table 11.

**Table 11: Glycerin Investigation**

| Lot No. | Batch Size | Propylene Glycol Concentration | Glycerin Concentration | Observation |
|---|---|---|---|---|
| 1 | 1.0 kg | 10% | 33% | Sink |
| 2 | 1.0 kg | 10% | 35% | Sink |
| 3 | 1.0 kg | 10% | 36% | Sink |
| 4 | 1.0 kg | 10% | 37% | ink |
| 5 | 1.0 kg | 10% | 38% | Sink |
| 6 | 1.0 kg | 10% | 39% | Suspension |
| 7 | 1.0 kg | 10% | 40% | Float |
| 8 | 1.0 kg | 10% | 41% | Float |

Based on the result of sample 6, the density was matched at the 39% glycerin (1.113g/mL theoretical) and all the subsequent batches had a glycerin concentration of 39%. The propylene glycol concentration of the formula was finalized at 10% w/w, and all the subsequent batches used 10% propylene glycol.

Fully neutralized samples from each of the above batches were transferred into centrifuge tubes in duplicate, 30 mL/tube. All samples were centrifuged at 2500 rpm for 5 hour at room temperature. Ibuprofen was than measured from the top and bottom of the centrifuge tube (Table 14).

**Table 12: Centrifugation study of neutralized Ibuprofen NSG with different levels of glycerin**

| Lot No. | Glycerin Concentration | Assay (mg/g) | | Difference (%) |
|---------|------------------------|------|--------|----------------|
| | | Top | Bottom | |
| 1 | 33% | 17.64 | 17.05 | -3.5 |
| | | 17.71 | 17.18 | -3.1 |
| 2 | 35% | 13.54 | 20.01 | 32.3 |
| | | 17.54 | 19.89 | -3.9 |
| 3 | 36% | 17.29 | 17.17 | -0.7 |
| | | 17.45 | 17.29 | -0.9 |
| 4 | 37% | 17.24 | 16.91 | -2.0 |
| | | 17.03 | 17.03 | 0 |
| 5 | 38% | 16.92 | 17.05 | 0.8 |
| | | 17.18 | 16.74 | -2.6 |
| 6 | 39% | 16.85 | 16.96 | 0.5 |
| | | 16.48 | 16.95 | 2.8 |
| 7 | 40% | 17.02 | 16.96 | -0.4 |
| | | 16.95 | 16.81 | -0.8 |
| 8 | 41% | 17.09 | 17.04 | -0.3 |
| | | 16.81 | 16.96 | -0.9 |

These results show that there is no significant difference in ibuprofen concentration between the top and bottom level of the centrifuge tube.

## EXAMPLE 9

The following amounts of poloxamer 188 and ibuprofen were weighed and placed on the undisturbed surface of 500 grams water. The time required to completely wet and sink the powder was measured.

**Table 13: Poloxamer 188 concentration study**

| Sample No. | % Ibuprofen (w/w) | % Poloxamer 188 in Water (w/w) | % Poloxamer 188 in Formula (w/w) | Time Required to Wet |
|------------|-------------------|-------------------------------|----------------------------------|----------------------|
| 1 | 1.74% | 0.00% | 0.00% | Unable to wet |
| 2 | 1.74% | 0.05% | 0.025% | About 24 hours |
| 3 | 1.74% | 0.10% | 0.05 % | About 7 hours |
| 4 | 1.74% | 0.20% | 0.10% | About 5 hours |

The ibuprofen was wetted in all three levels of poloxamer 188 samples. A suitable level of poloxamer 188 in the formula is 0.05% w/w.

## EXAMPLE 10

Sorbitol crystalline at about 5% w/w was used to facilitate the carbomer dispersion (data not shown). In order to optimize the viscosity of the gel, the carbomer vs. viscosity relationship was studied for the cherry flavor formula. Results are shown in Table 14.

**Table 14: Carbomer concentration - viscosity relationship**

| Sample | % Carbomer 934P | PH | Viscosity (mPa·s)[1] |
|---|---|---|---|
| 1 | 0.50% | 4.57 | 36660 |
| 2 | 0.40% | 4.73 | 23180 |
| 3 | 0.30% | 4.45 | 110370 |
| 4 | 0.28% | 4.71 | 8470 |
| Brookfield viscometer VIS-02 with spindle C at 20 rpm at room temperature (PD039, p26-29, 34-39) | | | |

Replacing a concentration of about 0.0015% cherry flavor with a concentration of about 0.0083%, resulted in a decrease of viscosity as shown in Table 15.

**Table 15: Effect of berry flavor on viscosity**

| Berry Flavor in the Batch | Viscosity (mPa·s)[1] |
|---|---|
| No | 8800 |
| Yes | 8100 |
| [1] Brookfield viscometer VIS-02 with spindle C at 20 rpm at room temperature | |

An experiment was conducted to investigate the effect of butylparaben on the viscosity of the ibuprofen suspension. It was concluded that viscosity decreased as the butylparaben was added to the batch. Results are shown in Table 16.

**Table 16: Effect of butylparaben on viscosity**

| Lot No | Butylparaben (%) | Visicosity (mPa·s)[1] | |
|---|---|---|---|
| | | 23 °C | 45 °C |
| 1 | 0 | 7570 | 1980 |
| 2 | 0.18 | 7100 | 1860 |
| [1] Brookfield viscometer VIS-02 with spindle C at 20 rpm | | | |

A carbomer concentration investigation was also conducted for an ibuprofen suspension containing cherry flavor. The following three 1.0 kg batches were compounded for carbomer concentration investigation. The concentration of butylparaben (0.018%) was recommended based on the levels measured in the Tylenol (Grape and Bubblegum suspensions). Results are shown in Table 17.

**Table 17**

| Lot No. | Butylparaben | Carbomer 934P | Viscosity (mPa·s)* |
|---|---|---|---|
| 1 | 0.018% | 0.40% | 7100 |
| 2 | 0.018% | 0.47% | 11030 |
| 3 | 0.018% | 0.45% | 10190 |
| 4 | 0.0% | 0.44% | 9130 |
| * VIS02, Spindle C, 20RPM at 23 °C | | | |

A carbomer concentration investigation was also tested for an ibuprofen suspension containing berry flavor. A 1.0 kg Ibuprofen NSG, was compounded. This batch contained 0.018% ofButylparaben and 0.47% of Carbomer. The viscosity of the batch was 10250 (mPa·s) at 23°C and 8520 (mPa·s) at 26.9°C. Results are shown in Table 18.

**Table 18**

| Lot No. | Butylparaben | Carbomer 934P | Viscosity (mPa·s) | Temperature |
|---|---|---|---|---|
| 1 | 0.018% | 0.47% | 10250 | 23°C |
| 2 | 0.018% | 0.47% | 8520 | 26.9°C |
| 3 | 0.0% | 0.46% | 9880 | 23°C |

The formulations of berry flavor were tested with and without the preservative butylparaben. The following Ibuprofen NSG Berry Flavor formulations were compounded:
1 1.0 kg, 0.0% butylparaben, 0.41% carbomer, viscosity 7200 (mPa·s)
2 1.0 kg, 0.018% butylparaben, 0.42% carbomer, viscosity 7870 (mPa·s)
3 1.0 kg, 0.018% butylparaben, 0.43% carbomer, viscosity 8070 (mPa·s)

The above batches were compounded for viscosity investigation. It was found that the specific gravity of the Ibuprofen NSG was different from the data obtained before. Therefore, the specific gravity was re-measured and the average data determined to be 1.11915 g/mL. Based on this result, the ibuprofen concentration was adjusted to 1.79%. The following two batches were compounded to evaluate the viscosity of the formula after changing the ibuprofen concentration.

**Table 19**

| Lot No. | Size | % Butylparaben | % Carbomer | Viscosity (mPa·s) |
|---|---|---|---|---|
| 1 | 1.0 kg | 0.018% | 0.43% | 8250 |
| 2 | 1.0 kg | 0.0% | 0.42% | 7570 |

To further investigate whether Carbomer 934P concentration can be adjusted to give the optimal viscosity. The following batches were compounded with different levels of carbomer 934P.

**Table 20 Carbomer - viscosity relationship for preservative formula**

| Lot No. | % Carbomer 934P | Flavor | Viscosity (mPa·s)[1] |
|---|---|---|---|
| 1 | 0.40% | Cherry | 7100 |
| 2 | 0.45% | Cherry | 10190 |
| 3 | 0.47% | Cherry | 11030 |
| 4 | 0.42% | Berry | 7870 |
| 5 | 0.43% | Berry | 8070 |
| 6 | 0.47% | Berry | 10250 |
| [1] Brookfield viscometer VIS-02 with spindle C at 20 rpm, 23 °C | | | |

Based on all of the above information, it was concluded that a suitable carbomer 934P concentration is 0.43% for berry flavor formula and 0.41% for cherry flavor formula. However, the physical properties might be different among different lots of carbomer 934P. This affects the final viscosity and the non-spill property of the formula. Therefore, depending on quality control, if a different lot of carbomer is used, then the concentration of the carbomer should be tested and reestablished.

**EXAMPLE 11**

The samples were subjected to a screen standard microbial test as required for assessment of preservative efficiency. Cultures were reviewed for growth of organisms after two weeks and four weeks. In all the batches, the bacteria showed a log reduction of not less than 2.0 from the initial count at 14 days and no increase from the 14 days count at 28 days. Yeast (C. albicans) and molds (A. niger) showed no increase from the initial calculated count at 14 and 28 days in all the above three batches. Therefore, these three batches meet requirements for the preservative challenge test. Preservatives are not required for the ibuprofen spill resistant suspension formula based on the results of the preservative challenge test.

## EXAMPLES 12-16

In the following examples the density of the ibuprofen ingredient is 1.11, sorbitol is 1.50, glycerin is 1.26, propylene glycol is 1.04 and water is 1.00. These examples provide variations in the amount of certain ingredients of the vehicle while maintaining the inventive integrity of the suspension. This is possible by balancing the amounts of components that have similar densities and calculating and maintaining the density of the vehicle components to match that of the active ingredient. For example, maximizing water while minimizing glycerin both having similar densities, and maximizing sorbitol while minimizing glycerin, both of which have high densities. The amount of propylene glycol is limited by the solubility it imparts to the ibuprofen.

Other ingredients are in low amounts and would have minimal impact on the system density. The organoleptic ingredients improve the taste and appearance and do not negatively affect the suspension stability. The carbomer is present to impart non-spill characteristics. The wetting agent is believed to contribute to the ease of processing but could be eliminated with the right processing conditions. The organoleptic agents in the following examples include coloring and flavoring agents, sweeteners and masking agents, and together with the ibuprofen make up about 4% of the formulations. These examples may contain a wetting agent (e.g., about 0.05% poloxamer 188), pH adjuster (e.g., about 0.08% sodium hydroxide), preservative (e.g., about 0.018% butylparaben), about 1.435% organoleptic agents, about 0.41% Carbomer and about 1.79% Ibuprofen.

### Table 21 Density Matching Vehicles

| Ingredient | Ex. 12 | | Ex. 13 | | Ex. 14 | | Ex. 15 | | Ex. 16 | |
|---|---|---|---|---|---|---|---|---|---|---|
| | Vol % | Weight | Vol % | Weight | Vol % | Weig ht | Vol % | Weig ht | Vol % | Weight |
| Water | 42.217 | (42.217) | 52 | (52) | 64 | (64) | 46 | (46) | 29 | (29) |
| Glycerin | 39 | (49.14) | 0 | | 12.4 | (15.6) | 50 | (63) | 47 | (59.2) |
| Sorbitol | 5 | (7.5) | 24 | (36) | 19.6 | (29.4) | 0 | | 0 | |
| Propylene glycol | 10 | (10.4) | 20 | (20.8) | 0 | | 0 | | 20 | (20.8) |
| | 96.217 | ( (96.257)) | 96 | ((94.8)) | 96 | ((94)) | 96 | ((93)) | 96 | ((92)) |

## Example 17

Stability testing was done at 0 months, 6 months, 12 months and 18 months post manufacturing of the Exhibit Batches. The following tests were done:

A. Description of physical properties of product.

B. The pH of the undiluted product was measured using pH-meter. (Target: 5.3. Limit: 5.0 to 5.6.)

C. Viscosity was measured using a Brookfield Viscometer with a 'T-C' spindle 20 RPM at 20-25°C.

D. The assay, impurity and preservative content was measured by the reverse-phase HPLC, using Phenomenex Luna C18 column with the mobile phase containing methanol/acetonitrile/phosphate buffer at pH 2.3 (120/360/520), eluting at 1.5 mL/min for 25 minutes, then 3.0 mL/min for 12 minutes for the column wash, and detection by UV absorption at 220 nm. Ibuprofen samples were prepared for analysis by dissolving about 5.5 grams of product in the sample solvent and its subsequent dilution in the sample solvent to desirable concentration. The typical retention time of preservative (butylparaben) is 6.76 minutes, ibuprofen 15.20 minutes and identified impurity (4-Isobutylacetophenone) 17.22 minutes.

E. Bottle uniformity is a measure of the Ibuprofen at the top, middle and bottom levels of the bottle. Amount of active was measured by HPLC as explained above (step D).

F. Amount of dissolved active (% label claim) was adopted directly from the Ibuprofen Suspension USP Dissolution procedure for ibuprofen oral suspension. Dissolution testing measured the amount of ibuprofen dissolved in 60 minutes in a dissolution apparatus using a spindle at 50 rpm (revolutions per minute) at 37°C. A dissolution apparatus (Distek Inc., North Brunswick, NJ), equipped with 6 to 8 vessels immersed in a water bath and maintained at 37±0.5°C. Individual spindles for each vessel were employed for the testing. Samples were taken by syringe at regular intervals from each vessel and measured by HPLC.

Results are given in Table 22.

**Table 22: Stability Data**

|  | 0 Months | 6 Months | 12 Months | 18 Months |
|---|---|---|---|---|
| **Description** | Orange, opaque, viscous, jellylike material with characteristic berry odor | Orange, opaque, viscous, jellylike material with characteristic berry odor | Orange, opaque, viscous, jellylike material with characteristic berry odor | Orange, opaque, viscous, jellylike material with characteristic berry odor |
| **pH** | 5.3 | 5.3 | 5.2 | 5.2 |
| **Viscosity** | 8320 mPa·s | 8000 mPa·s | 6900 mPa·s | 7900 mPa·s |
| **Ibuprofen Assay (mean of Bottle Uniformity)** | 99.1% | 101.5% | 101.9% | 102.1% |
| **Bottle Uniformity (with relative standard deviation)** | Top: 99.1% Middle: 99.0% Bottom: 99.2% %RSD 0.1% | Top: 101.5% Middle: 102.6% Bottom: 101.9% %RSD 1.3% | Top: 100.6% Middle: 101.8% Bottom: 103.2% %RSD 1.3% | Top: 98.9% Middle: 102.2% Bottom: 105.2% %RSD 3.1% |
| **Butylparaben Assay** | 103.1% | 104.4% | 104.6% | 103.4% |
| **Dissolution** | $V_1 = 96\%$ $V_2 = 96\%$ $V_3 = 96\%$ $V_4 = 97\%$ $V_5 = 102\%$ $V_6 = 99\%$ | $V_1 = 102\%$ $V_2 = 102\%$ $V_3 = 102\%$ $V_4 = 100\%$ $V_5 = 101\%$ $V_6 = 101\%$ | $V_1 = 102\%$ $V_2 = 102\%$ $V_3 = 102\%$ $V_4 = 102\%$ $V_5 = 102\%$ $V_6 = 102\%$ | $V_1 = 102\%$ $V_2 = 102\%$ $V_3 = 102\%$ $V_4 = 102\%$ $V_5 = 102\%$ $V_6 = 102\%$ |
| **Microbiological Examination** | Less than 10 microorganisms/g (no Salmonella or E.Coli) | NA | NA | NA |
| **Degradation Products** | | | | |
| 4-Isobutylacetophenone | Not detected | Less than 0.050% | Not detected | Not detected |
| Individual Unidentified | Less than 0.050% | Less than 0.050% | Less than 0.050% | Not detected |
| Total | Less than 0.050% | Less than 0.050% | Less than 0.050% | Not detected |

The same procedure will be done on samples from an exhibit batch at 24, 30 and 36 months post manufacturing. It is expected that there will be no change in the stability data at the future dates. Based on the data from the first eighteen months of stability testing, the pH, viscosity, bottle uniformity, dissolution, microbiological examination, active agent and degradation products will remain stable throughout the shelf-life of the batch.

**Claims**

1.  A suspension comprising (w/w) 0.5 to 5% ibuprofen, up to 1% organoleptic agents, from 0.4 to 0.5% carbomer 934P, from 5 % to 10% sorbitol crystalline, from 10% to 20% propylene glycol, from 33% to 41% glycerin, and up to 0.4% sucralose liquid concentrate, wherein the carbomer 934P has been neutralized to a pH range of 4.8 to 5.6.

2.  The suspension of claim 1, comprising 1.79% ibuprofen.

3.  The suspension of claim 1, further comprising 42% water, 39% glycerin, 5% sorbitol and 10% propylene glycol.

4.  The suspension according to claim 1, comprising 1.79% ibuprofen (w/w), 0.48% to 0.50% (w/w) carbomer 934P, 0.08% (w/w) sodium hydroxide, 0.05% (w/w) poloxamer 188, 10.0% (w/w) propylene glycol, 39.0% (w/w) glycerin, 5.0% (w/w) sorbitol crystalline, 0.40% (w/w) sucralose liquid concentrate, 0.005% (w/w) food and drug color yellow number 6, 0.20% (w/w) masking agent, 0.83% berry flavor, and 42% purified water.

5.  The suspension of claim 4, further comprising up to 0.18% (w/w) butylparaben.

6.  The suspension of claim 4, further comprising up to about 0.04% (w/w) propylparaben.


**Patentansprüche**

1.  Eine Suspension umfassend (w/w) 0,5 bis5%Ibuprofen, bis zu 1%organoleptische Wirkstoffe, von 0,4 bis 0,5%Carbomer 934P, von 5%bis 10%kristallines Sorbitol, von 10%bis 20%Propylenglykol, von 33%bis 41%Glycerin und bis zu 0,4%flüssiges Sucralosekonzentrat, wobei das Carbomer 934P auf einen pH-Bereich von 4,8 bis 5,6 neutralisiert wurde.

2.  Die Suspension nach Anspruch 1 umfassend 1,79%Ibuprofen.

3.  Die Suspension nach Anspruch 1 ferner umfassend 42%Wasser, 39%Glycerin, 5% Sorbitol und 10%Propylenglykol.

4.  Die Suspension nach Anspruch 1 umfassend 1,79%Ibuprofen (w/ w), 0,48%bis0,50% (w/ w) Carbomer 934P, 0,08% (w/w) Natriumhydoxid, 0,05%(w/w) Poloxamer 188, 10,0%(w/w) Propylenglykol, 39,0%(w/ w) Glycerin, 5,0%(w/w) kristallines Sorbitol, 0,40%(w/w) flüssiges Sukraiosekonzentrat, 0,005%(w/w) gelbe Lebensmittel- und Arzneimittelfarbe Nummer 6, 0,20%(w/ w) Maskierungsmittel, 0,83%Beerenaroma und 42%gereinigtes Wasser.

5.  Die Suspension nach Anspruch 4 ferner umfassend biszu 0,18%(w/w) Butylparaben.

6.  Die Suspensions nach Anspruch 4 ferner umfassend biszu etwa 0,04%(w/w) Propylparaben.


**Revendications**

1.  Suspension comprenant (en poids) 0,5 à 5% d'ibuprofène, jusqu'à 1% d'agents organoleptiques, de 0,4 à 0,5% de carbomère 934P, de 5 % à 10% de sorbitol cristallin, de 10% à 20% de propylène glycol, de 33% à 41% de glycérine, et jusqu'à 0,4% de concentré liquide de sucralose, dans laquelle le carbomère 934P a été neutralisé à une gamme de pH de 4,8 à 5,6.

2.  Suspension selon la revendication 1, comprenant 1,79% d'ibuprofène.

3.  Suspension selon la revendication 1, comprenant en outre 42% d'eau, 39% de glycérine, 5% de sorbitol et 10% de propylène glycol.

4.  Suspension selon la revendication 1, comprenant 1,79% d'ibuprofène (en poids), 0,48% à 0,50% (en poids) de carbomère 934P, 0,08% (en poids) d'hydroxyde de sodium, 0,05% (en poids) de poloxamère 188, 10,0% (en poids) de propylène glycol, 39,0% (en poids) de glycérine, 5,0% (en poids) de sorbitol cristallin, 0,40% (en poids) de concentré liquide de sucralose, 0,005% (en poids) de colorant jaune numéro 6 alimentaire et pharmaceutique, 0,20% (en poids) d'agent de masquage, 0,83% d'arôme de baie, et 42% d'eau purifiée.

**5.** Suspension selon la revendication 4, comprenant en outre jusqu'à 0,18% (en poids) de butylparabène.

**6.** Suspension selon la revendication 4, comprenant en outre jusqu'à environ 0,04% (en poids) de propylparabène.

Figure 1

Figure 2

**Figure 3**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6071523 A, Mehta
- US 6102254 A, Ross
- US 4788220 A, Mody
- US 5374659 A, Gowan, Jr.
- EP 0479005 A

**Non-patent literature cited in the description**

- McGraw-Hill Encyclopedia of Science & Technology. 1987, vol. 12, 57-60
- **CARBOMERS.** Carbomers
- Handbook of Pharm. Excipients. Handbook of Pharmaceutical Excipients. Pharmaceutical Press, 2000, 442, 79, 53
- **B.F.Goodrich.** *Company The Merck Index,* 1996, 839
- Pharm. Dosage Forms: Disperse System. Pharmaceutical Dosage Forms: Disperse System. Marcel Dekker, Inc, 1988, vol. 1, 181
- Pharmaceutical Dosage Forms: Disperse System. Marcel Dekker, Inc, 1996, vol. 2, 152
- *Pharm. Dosage Forms,* vol. 2